# EUROPEAN PATENT SPECIFICATION

(11) **EP 1 534 169 B1**
(45) Date of publication and mention of the grant of the patent: **08.03.2006**
(21) Application number: 03794348.7
(22) Date of filing: 03.09.2003
(51) Int. Cl.: A61C 13/00, A61C 13/08, A61K 6/06, C04B 41/50

(54) **STRENGTHENED CERAMIC RESTORATION**
VERSTÄRKTE KERAMIKRESTAURATION
RESTAURATION UTILISANT UNE CERAMIQUE RENFORCEE

(30) Priority: 05.09.2002 EP 02078708
(43) Date of publication of application: 01.06.2005
(73) Proprietor: Elephant Dental B.V., 1628 PM Hoorn (NL)
(72) Inventor: VAN DER ZEL, Joseph, Maria, NL-1628 TN Hoorn (NL); SLOR, Jan, NL-1394 BK Nederhorst den Berg (NL); GRINWIS, Theodorus, Jacobus, NL-1611 JE Bovenkarspel (NL); DE KLER, Marcel, André, NL-1815 SC Alkmaar (NL); TSADOK HAI, Tsadok, NL-1613 EP Grootebroek (NL); KREUDER, Peter, 61231 Bad Nauheim (DE)
(74) Representative: Winckels, Johannes Hubertus F.
(86) International application number: PCT/NL2003/000616
(87) International publication number: WO 2004/021921

(56) References cited:
- EP-A- 0 218 853
- EP-A- 0 631 995
- US-A- 5 833 464
- US-A1- 2002 006 532

## Description

The present invention relates to an aesthetic ceramic dental restoration, and particularly to a crown, part-crown or a bridge. In addition, the invention relates to a process for manufacturing such a product. More specifically, the present invention concerns a process for the production of an aesthetic heat-pressed restoration in occlusion, which is strengthened by a zirconia suprastructure, as well as the product obtainable in this way.

Dental restorations are mostly metal-ceramic composite structures, comprising a metallic framework used for load bearing, and ceramic or porcelain coatings for aesthetic appearance. More in detail, nowadays, about 80% of all fixed restorations are metal-ceramic restorations; these metal ceramic restorations show a clinical survival rate of at least 95% after 7.5 years.

In the vast majority of manufacturing processes, the ceramic material is applied by using repeated layer formation. Particularly, a layer of ceramic or porcelain is applied, followed by firing or sintering, which steps are repeated until the suitable dental restoration is obtained. During this conventional process, each consecutive layer of porcelain shrinks during sintering. This shrinkage makes it very hard to obtain proper occlusal contacts with the antagonist teeth.

It would be very desirable to find a cost and time saving process compared to the traditional porcelain layering process.

Another disadvantage of the conventional layering technique is that often defects such as bubbles or clefts are formed, having an adverse effect on the adhesion between porcelain and either translucent layer or core or support material.

In the prior art, attempts have been made to solve or reduce these problems. In this light, reference can be made to an article titled "Gießen, pressen, modellieren" published in DZW (Woche) 23/02. In this reference, a process is described for preparing a dental restoration in occlusion, wherein a cast metal structure is first covered with two opaque liner layers, followed by waxing-up up till the occlusal contacts with the opposing teeth and mesio-distal contacts with the neighbouring teeth are obtained. Sprues are attached to the wax part, the restoration is embedded in a refractory material and a tooth coloured glass ceramic is pressed onto the metal prestructure. Because of the non-translucent nature of the metal, the metal shimmers through the translucent outer glass ceramic which results in an less attractive looking restoration.

Beside the fact that these known metal-ceramic combinations show limitations concerning aesthetic appearance, the last years, the use of metals in the oral cavity has been disputed due to their biological incompatibility risk. This led to the need for bio-inert, metal free dental restorations.

In the art, it was proposed to meet this need by making all-ceramic dental restorations or prostheses. Such all-ceramic dental restorations have been made of feldspathic porcelain, leucite re-inforced porcelain, alumina, glass-infiltrated porous alumina and glass ceramics. These ceramic materials show, however, low bend strengths and toughness, which properties imply design restrictions, non-reliability and complicated multistep manufacturing procedures for, e.g., dental bridges.

Based on these findings, the person skilled in the art has focussed its attention to zirconia base materials to replace the metal base, because zirconia has promising properties in respect of durability and longevity. Reference can be made to the article of Filser *et al.* titled "All-Ceramic Dental Bridges by Direct Ceramic Machining (DCM)" as published in: Materials in Medicine, Materials Day, Department of Materials, Eds. M.O. Speldel; P.J. Uggowitzer; vdf Hochschulverlag AG, ETH Zürich; Zürich (1998) 165-189 Zirconia structures can be produced by slib casting or by milling. Because of the opaqueness of zirconia, a tooth-coloured dental glass is used to bring the proper natural aesthetics to the zirconia base.

The zirconia used is mostly partially stabilized zirconia, and more in detail tetragonal zirconia stabilized with yttria, which has high strength and toughness.

The starting powder for these ceramic restorations has special demands for particle size and morphology as described in detail in the article of Filser *et al*.. These demands are necessary for an homogeneous result on the consolidation by isostatic pressing at a pressure higher than 2000 bar. Blocks are subsequently partially sintered at 900°C until the powder particles are bonded by neck growth to give the ceramic body strength high enough to be able to mill it. Milling is done taking account of an enlargement factor that corresponds to the expected sintering shrinkage in the final sintering step. The zirconia structure is then coated with a veneer porcelain to meet the patient's requirements concerning colour and translucency.

In EP-A-0 631 995, all-ceramic restorations are prepared by pressing and heating a combination of 50-99 wt.% ceramic and 1.50 wt.% glass in a mold. Although it is preferred in the invention of EP-A-0 631995 to use alumina powder and/or zirconia powder as ceramic, also yttrium stabilized zirconia is mentioned. Further, it is indicated that the aesthetic character of the prosthesis can be improved by veneering the ceramic/glass composite. The veneering composition is not applied by pressing, nor described in detail.

Although the traditional layering technique could be used, thermal pressing of dental glass into a lost wax form is a more effective and economic way.

EP-A-0 218 853 discloses YTZP sintered in two stages in oxidising atmosphere where the second stage is by hot isostatic pressing. To create a coloured caramic, a colourant, an inorganic pigment can be added to the sintered material. The ceramic can be used for tooth crowns.

US patent application No. 2002/006532 discloses a YTZP dental component. The grain size is less than 0.5 µm. The ceramic is so treated as to form a glossy phase and bonding surface.

Cornelissen gives in TTM: Magazine voor Tandartsen en Tandtechnici 10 (2001) and in Quintessenz Zahntech. 28(2) (2002). 150-158, a description of the Cordent crown. This crown is prepared by directly modelling the entire dentine form inclusive crown shoulder to an AGC Galvano yellow cap, embedding in refractory, pressing ceramic, and debedding, followed by divesting and glazing firings- The yellow metal cap is said to provide a nice deep orange glow at the occlusal and near the edges. Cornelissen notes that in the Cordent crown the advantages of all-ceramic systems are combined with the advantages of metal-ceramic systems.

Processes are described whereby a wax model is embedded in a refractory material and after hardening of the mould the wax is burnt-out. A glass material in the form of a dense pellet is brought in over the pressing connector channels and with a refractory cylinder under thermal plastification the pellet is pressed in the mould.

These materials lack sufficient strength to be used for more stressed applications such as bridges,

In accordance with the present invention full-ceramic dental restorations are prepared which meet all needs sketched combined with the advantages of the described prior art systems. Other advantages and benefits of the present invention will become clear after reading the following description.

More in detail, the present invention relates to a process for the preparation of a full-ceramic dental restoration, which should be in occlusal contact with opposing teeth and in mesio-distal contact with neighbouring teeth, comprising heat pressing of a tooth coloured pressing glass on a fully or partially supporting structure of yttria doped tetragonal zirconia (YTZP), comprised of yttria doped tetragonal zirconia ceramic having a grain size, as measured by the linear intercept method, of less than 0.6 µm, wherein the thermal expansion coefficient (TEC) of the pressing glass lies between 9.0 and 11.0 µm/m.K (measured in the range of from 25 to 500°C) and the pressing temperature of the pressing glass lies between 750 and 1000°C.

In a further aspect, the present invention relates to a full-ceramic dental restoration, comprising a fully or partially supporting structure of yttria doped tetragonal zirconia (YTZP) and a heat pressed tooth coloured pressing glass, obtainable by the process of the invention. Preferably, this restoration is modelled to be in occlusal contact with opposing teeth and in mesio-distal contact with neighbouring teeth.

Surprisingly, it was found according to the present invention that a tooth coloured glass ceramic when pressed on a strong zirconia structure, which has a certain degree of translucency, a very naturally looking restoration can be obtained, even when no liner was used. The zirconia used is referred to in the above-mentioned article of Filser *et al*., and described in more detail by Lüthy in W.H. Mörmann (ed.), CAD/CIM in Aestetic Dentistry, Qumtessenz, Chicago, (1996), 229 ff., and has a high strength and can be used for support structures in single element restorations as well as in larger constructions such as 3 to 4-unit bridges.

The ability to heat-press a tooth coloured glass ceramic onto such a structure means an enormous time saving when compared to the layering of porcelain powder and subsequently sintering the powder as is usual in the traditional way. This cost and time saving is in comparison to the tradition porcelain layering process. During this latter process each consecutive layer of porcelain shrinks during sintering making it very hard to obtain proper occlusal contacts with the antagonist teeth.

Because the press-pellet is already in a tooth colour, the colour of the restoration will not vary from the given colour as can be the case when layering with a variety of colours. The colouring of the porcelain used in the present invention is known to the person skilled in the art. A suitable method is described in detail in DE-OS-199 04 522, which document is incorporated by reference in the present description for describing the method of colouring.

It is for instance possible to apply pure oxidic pigments on the zirconia, followed by the sintering together of the zirconia and oxidic pigments. In a particular embodiment, the pigments are pressed with a binder into a block or cylinder to be used as a pensil to bring the pigments on the zirconia surface.

In another embodiment, the partially sintered zirconia structure is impregnated with a solution of metal chorides, nitrates, acetates or alcoholates and, subsequently dried and sintered to obtain a tooth coloured zirconia structure after sintering. Very suitable results are obtained, while using metals of the group of iron, praesodimium, nickel, cerium, erbium, cobalt, and copper.

In a preferred embodiment, the supporting structure is densely sintered; preferentially, the ceramic is produced by CAD/CAM technology. More specifically, in a preferred embodiment of the process of the invention the ceramic is milled by a CAD/CAM-system in the green state or in a partially sintered state, followed by sintering to full density.

In the process of the invention very good results are obtained with a supporting structure formed from electrophoretic deposition of zirconia from a slurry, followed by sintering to full density.

In another embodiment, the structure is milled out of an hot isostatically hopped zirconia.

Apart from the above, the German "Patentschrift" 196 30 412 and the corresponding US-A-5,833,464 teach a process for the fabrication of a full-ceramic dental build-up on a zirconia root pin, wherein a zirconia-glass is heat-pressed against the root pin that has a TEC that is the same or up to 3.0 µm/m.K higher than the TEC of the zirconia glass. These prior art documents also describe the build-up with zirconia glass of a tooth replacement without mentioning how the zirconia is used. No reference is made to occlusal restorations, as is the subject of the present invention. Moreover, the zirconia glass described in the German and U.S. patent has low transparency and cannot be used for aesthetic tooth-like restorations, as is the case with the present invention.

The process of the invention directly presses the occlusal contacts in an aesthetically working material after the occlusal contact with the antagonistic teeth and the mesio-distal contacts with the neighbouring elements having been precisely modelled in wax in the traditional way.

Another advantage over the traditional layering is the high density, low defect structure that can be obtained by pressing a densily sintered glass instead of applying a porcelain as a powdery substance with subsequent sintering. The latter shows very seldom a bubble-free structure.

Another advantage over the traditional layering is the adhesion obtained between the translucent material and the zirconia core. During layering the boundary layer often shows defects such as bubbles and clefts, while the pressed on glass shows an excellent defect free boundary, resulting in a better adhesion and a higher structural strength.

For particular embodiments, advantages can be obtained when a conventional liner is applied to the supporting zirconia structure which liner has a melting point less that 50°C lower than the pressing temperature of the pressing glass.

The process offers another advantage in the possibility of directly pressing a shoulder with a perfect fitting margin, without showing the substructure at the edge of the supporting material. For this the edge of the supporting core is kept short by 0.5 to 2 mm from the edge.

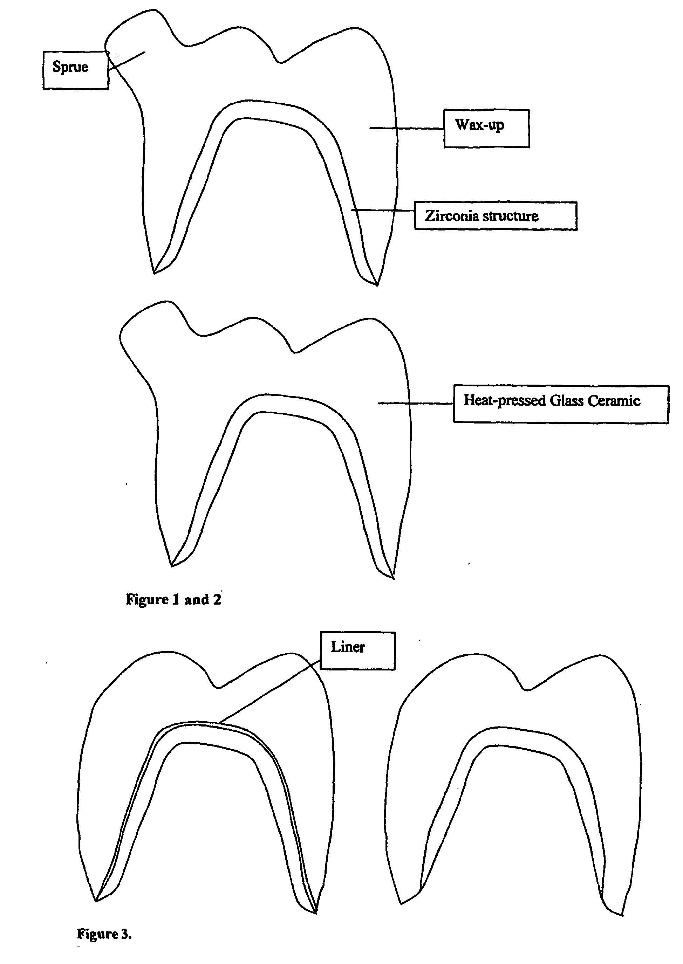

The process of the invention has the advantage of the possibility to create a chameleon effect, when using the heat press ceramic as shoulder material (Figure 3; Right). Because the wax has been applied after the zirconia structure is fitted on the gypsum die, the wax-up for the shoulder can follow the die exactly around the margin, and the subsequently pressed shoulder reproduced in glass ceramic will have the same good fit. In the traditional way involving several steps of layering porcelain, several corrections with porcelain additions have to be made to produce a fitting margin, because of the shrinkage of the porcelain powder during sintering.

In a particular aspect of the process of the invention, the structure is, hence, kept short by 0.5 to 2.0 mm from the edge of the final restoration to be made, after which the pressing glass is pressed in such a way that it forms an aesthetically pleasing shoulder without showing the core at the margin.

Preferably, the stresses are transferred on the core only, while leaving the shoulder free from the prepared tooth.

The present inventors have found that the stability of the adhesive bond strength between the glass and the zirconia is critically dependent upon the susceptibility of the zirconia material to low temperature degradation (LTD). Although YTZP zirconia ceramics are known to have a high strength and toughness, they are also known to be susceptible to strength degradation upon exposure to steam in the temperature range of about 100-500°C. The origin of this LTD phenomenon is attributed to a reaction involving water and the Zr-O-Zr bonds of the ceramic. This reaction causes transformation of zirconia grains from their desired tetragonal state to the monoclinic state. This transformation is accompanied by a volume expansion in the transformed grain of about 4 vol.%, which causes microcracking in the component and, accompanied, strength degradation.

Without wishing to be bound by any theory, it is believed that the environmental conditions present in the mouth are such that LTD may occur in the zirconia dental components and that this phenomenon may have a negative impact on the strength of the YTZP-dental glass bond. In particular, the temperatures in the mouth are typically simulated by thermal cycling between about 5 and 55°C. Although these temperatures are somewhat below those typically associated with the LTD phenomenon, Chevalier *et al.* (see: Bioceramics 10 Ed. L. Sedel and C. Rey (Proc. of the 10^{th} Int. Symp. on Ceramics in Medicine, Paris, France, October 1997) Elsevier Science Ltd.) have suggested that LTD may also occur in some YTZP zirconias at temperatures as low as about 37°C. Thus, it is believed that LTD may act upon YTZP in dental systems. With regard to YTZP-glass bonds, it is believed that LTD of the YTZP may cause general microcracking in the vicinity of the transformed grain and in particular at the uncracked surface of YTZP material, and that this microcracking degrades the adhesive bond strength of the glass-zirconia system and allows for further ingress of water into the zirconia material, thereby accelerating the spread of LTD.

The selections made in the process of the present invention prevent or at least inhibit or reduce LTD.

In said article of Chevalier *et al*. suitable yttria-stabilised zirconias for use in the present invention are identified.

Very good results are obtained in a process, wherein the connector to the press pellet reservoir and the specimen is formed by a continuous flow plate of 1.5 to 2.5 mm thick. This embodiment will be described in more detail in Figure 8 and the accompanying text.

The present invention will now be described in more detail, wherein reference is made to the drawings, wherein:
Figure 1 shows a schematic overview of a zirconia structure with wax-up;
Figure 2 shows the zirconia structure of Fig 1 after pressing;
Figure 3 shows final restorations with a liner and with a zirconia shoulder (prior art) (left) and with a shoulder of heat-pressed ceramics in accordance with the present invention (right);
Figure 4 shows a flow scheme for the production of zirconia structures to be used in the present invention;
Figure 5 shows a flow scheme for the production of the pressing step wherein pressing glass is brought on the zirconia structures according to the present invention;
Figure 6 shows a microphotograph of a dental glass pressed to YTZP zirconia in the absence of a liner;
Figure 7 shows a microphotograph of a dental glass pressed to YTZP zirconia covered with a liner B; and
Figure 8 shows a special flow plate for a pressed glass structure.

References : DE 19904522 A1 Offenlegungsschrift ESPE Dental AG, Chevalier et al., Bioceramics 10:135-138 (1997) DE 19630412 C2 lvoclar Roat pin, Filser et al, DCM Process.

All of the above mentioned advantages are provided by the present invention which comprises the process of producing an esthetic heat-pressed restoration in occlusal and mesio-distal contact with neighbouring and opposing teeth, strengthened by a fine grained yttria doped tetragonal zirconia (YTZP) support structure.

The new glass composition that was produced has a pressing temperature from about 750 to 1000°C, and preferably form 900 to 950°C and has a coefficient of thermal expansion of from about 9.0 to 11.0, and preferably from about 9.0 to 10.0 x10⁻⁶/°C (25°C to 500°C).

More specifically, the present inventors have found a low expansion glass or preferably a glass-ceramic material suitable for over-pressing a zirconia suprastructure such as a crown, part-crown or bridge has been developed.

Taking into account the guidances given herein above, the glass used in the process of the invention preferably has the following chemical composition: 7-15 wt.% Al₂O₃, 13-23 wt.% of (K₂O + Na₂O), 1-3 wt.% of (BaO + CaO), 1-3 wt.% (Sb₂O₃ + Li₂O), and 0.2-1.2 wt.% fluor, the balance being SiO₂, and colouring compositions. In a more preferred embodiment, the glass has the following chemical composition: 7-15 wt.% Al₂O₃, 6-14 wt.% K₂O, 5-11 wt.% Na₂O, 0.2-2.5 wt.% BaO, 0.1-1.5 wt.% CaO, 1.2-2.5 wt.% Sb₂O₃, 0.05-0.5 wt.% Li₂O, and 0.5-1.0 wt.% fluor, the balance being SiO₂, and colouring compositions.

Low expansion glasses for use in the present invention can be produced by blending powdered metal oxides or carbonates or nitrates in the appropriate proportions. The blended powders are fused to form a glass melt followed by quenching, drying, ballmilling and seeving by means known in the art.

The powder formed from these glasses have a particle size of preferably less than 106 µm; they are pigmented to obtain a toothlike appearance. Then the powder is granulated with a binder and uniaxially dry-pressed at room temperature and then sintered at a temperature of 800° to 1000°C., preferably 900° to 960°C, for 1 minute to 1 hour, preferably 1 minute to 30 minutes.

The glass-ceramic pellet obtained in this way can then be over-pressed on a zirconia suprastructure embedded in the mould to obtain a restoration in occlusal contact with the opposing teeth and mesio-distal contacts with neighbouring teeth. A suitable embedding material is silica-based refractory such as Carrara® Press Speed (ex Elephant Dental B.V., Netherlands).

The zirconia supporting structure is prepared from yttria doped tetragonal zirconia. This stabilized zirconia should have a grain size, as measured by the intercept method, of less than 0.6 µm. Very good results are obtained when using a partially stabilized zirconia with a density of more than 99.0 wt.%, and preferably more than 99.5%, such as 99.8% of the theoretical density with an open porosity of less than 0.4% and preferably less than 0.2%. The elasticity modulus was not higher than 220 GPa and the fracture toughness was at least 5 MPa.m^{½}.

Not suitable were partially stabilized zirconias (PSZs) such as magnesia stabilized zirconias (Mg-PSZ) or calcia-stabilized zirconias (Ca-PSZ). These zirconias are characterized by a coarsely grained structure (of the order of about 50 µm) containing significant residual intragranular porosity. In addition, due to the high sintering temperature needed to sinter PSZs, impurity diffusion at the grain boundaries often produces a significant glassy phase, thereby affecting mechanical properties. The weak intergranular bonds of the coarse PSZ grains are more easily broken during adhesion testing, thereby lowering overall bond strength. In contrast, the very fine microstructure of biomedical grade YTZP materials results in very strong intragranular bonding. For this reason YTZP appeared a material providing a surface suitable for bonding to heat-pressed glass. Preferably, the YTZP ceramic has a grain size of less than 0.5 µm.

If desired, the zirconia structure can be coloured by ionic or complex containing solutions which contain rare earth elements or elements of the adjacent group. The partially sintered zirconia structure can for instance be dipped in such a solution, dried and sintered to its final density.

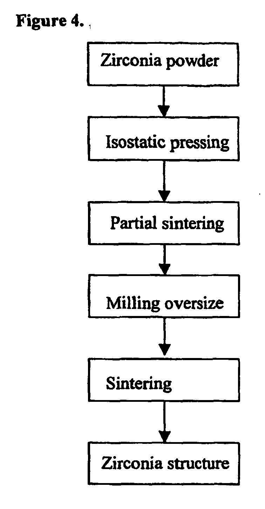

Figure 4 shows a production flow scheme for the zirconia structures for use in the present invention. Particularly, the zirconia powder is subjected to isostatic pressing, preferably using CAD/CAM technology. The pressed form is subjected to partial sintering and subsequently milled, although it can also be milled in green state, preferably using a CAD/CAM system. After milling the oversize, the formed structure is sintered to full density giving the zirconia substructure.

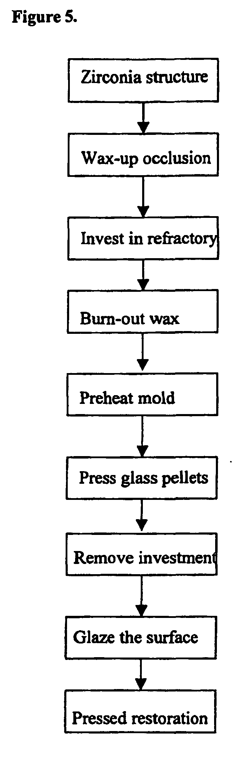

This substructure is further treated following the scheme shown in figure 5. The substructure and a wax-up occlusion (see Figure 1) are invested in refractory. The wax is burnt out, and the mould is preheated. Glass pellets are pressed in the mould, after which the investment is removed (see Figure 2). The product formed can be subjected to a treatment to glaze its surface.

In Figure 3, two final restorations of the present invention are described. The left-hand image shows a zirconia shoulder structure coated with a liner on which a low expansion glass of the invention is pressed. Thereto a suitable liner, for instance one consisting of 54.8 wt.% SiO₂, 12.9 wt.% Al₂O₃, 11.5 wt.%K₂O, 8.7 wt.% Na₂O, 10.4 wt.% CeO₂, 1.0 wt.% Li₂O and 0.4 wt.% B₂O; or one consisting of 58.5 wt.% SiO₂, 12.6 wt.% Al₂O₃, 11.0 wt.% K₂O, 7.1 wt.% Na₂O, 10.4 wt.% CeO₂, 0.4 wt.% LiO₂, was applied in a single coat of 20 to 40 µm onto a densely sintered zirconia support-structure and fired at 800°C and 915°C for both liners illustrated respectively.

The liner-coated zirconia coping was waxed up and sprued as described before. The substructure comprising the liner was subsequently overpressed with the glass material.

The right-hand image of Figure 3 shows a zirconia substructure with a shoulder in heat-pressed glass ceramics.

It was additionally found that when a special flow plate (fig 8) was used to guide the flowing heat-pressed glass a better filling was obtained with less material, that with separate sprues as is usual in the traditional technique. The plate is 1.5 to 2.5 mm thick and starts from one side with the press pellet reservoir and is over its full length in contact with the pressed specimen. The flow plate is easier to separate with the appropriate cutting wheels than the much thicker separate sprues in the traditional technique. The plate makes contact with the pressed specimen in a place where it does not interfere with the contact surfaces to the antagonistic teeth and the mesio-distal contact areas.

The present invention will be illustrated in more detail in the following, non-limiting examples. Where reference is made to percentages, weight percentages drawn to the weight of the final composition are meant, unless otherwise indicated.

### Examples 1-4

Low expansion glasses or glass-ceramic materials suitable for over-pressing a zirconia suprastructure such as a crown, part-crown or bridge were prepared. Thereto, four mixtures were produced by blending powdered metal oxides or carbonates or nitrates in the appropriate proportions. The blended powders were fused to form a glass melt followed by quenching, drying, ballmilling and seeving by means known in the art (see Table 1 for the final compositions).

The powder formed from either one of the four undermentioned glasses having a particle size of less than 106 µm are pigmented to obtain a toothlike appearance. Then the powder was granulated with a binder and uniaxially dry-pressed at 900°C for 20 minutes.

A partially stabilized zirconia with a density of 99.8% of theoretical density with an open porosity of less than 0.2% was obtained by following the method depicted in figure 4. The elasticity modulus was about 200 GPa and the fracture toughness was about 5 MPa.m^{½}.

The glass-ceramic pellets obtained as described were over-pressed on the zirconia suprastructure embedded in a mould to obtain a restoration in occlusal contact with the opposing teeth and mesio-distal contacts with neighbouring teeth.

**Table 1. Composition of heat-press glass ceramic**

| Component | Ex. 1 | Ex. 2 | Ex. 3 | Ex. 4 |
|---|---|---|---|---|
| SiO₂ | 66.2 | 65.0 | 67.4 | 66.0 |
| Al₂O₃ | 10.7 | 7.5 | 13.9 | 14.4 |
| K₂O | 9.5 | 12.2 | 6.8 | 9.1 |
| Na₂O | 8.5 | 9.6 | 7.4 | 6.8 |
| BaO | 1.2 | 2.0 | 0.4 | 0.4 |
| CaO | 0.7 | 0.2 | 1.3 | 1.1 |
| Sb₂O₃ | 2 | 2.3 | 1.8 | 1.5 |
| Li₂O | 0.2 | 0.4 | 0.1 | 0.2 |
| F | 0.8 | 0.9 | 0.7 | 0.6 |
| Pressing temperature, °C | 900 | 800 | 940 | 930 |
| Thermal Coefficient of Expansion (TCE), x10⁻⁶/°C (25°C to 500°C) | 9.5 | 11.0* | 8.7 | 10.1 |
| Break strength of incisor tooth, N (without liner) | 5200 | 5800 | 6300 | 6200 |
| Break strength of incisor tooth, N (with liner B) | N.D. | N.D. | N.D. | 6000 |
| Thermal Shock Tests, nr. of cycles up to 20 until cracks appear | 20 | Cracks | Cracks | 20 |

| | | | | |
|---|---|---|---|---|
| * TCE was measured from 25°C to 400°C | | | | |
| N.D. = not determined | | | | |

A liner material A, consisting of 54.8 % SiO₂, 12.9 % Al₂O₃, 11.5 %K₂O, 8.7 % Na₂O, 10.4 % CeO₂, 1.0 % Li₂O and 0.4 % B₂O, was applied in a single coat of 20 to 40 µm onto a densely sintered zirconia support-structure and fired at 800°C.

The liner-coated zirconia coping was waxed up and sprued as described before.

Upon overpressing a zirconia-structure embedded in a silica-based refractory form (Carrara® Press Speed, Elephant Dental B.V., Netherlands) with densely sintered pellets having the oxidic composition of Ex. 4, the liner layer melted and dripped to the margin area.

Another liner material B, consisting of 58.5 % SiO₂, 12.6 % Al₂O₃, 11.0 % K₂O, 7.1 % Na₂O, 10.4 % CeO₂, 0.4% LiO₂ was applied in a single coat of 20 to 40 µm and fired at 915°C. After the liner was overpressed using the same pellets as described above testing the liner material A, the layer remained in place and good results were obtained regarding both the thickness of the liner over the whole surface of the zirconia coping as for a good esthetic appearance.

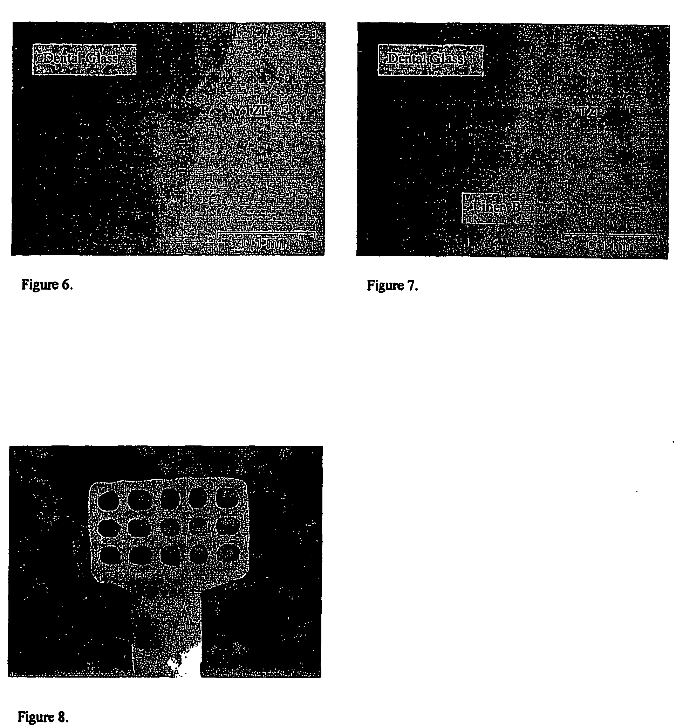

The interfaces formed were studied in cross section. The results are shown by the microphotographs in Figures 6 and 7.

## Claims

1. Process for the preparation of a full-ceramic dental restoration, comprising heat pressing of a tooth coloured pressing glass on a fully or partially supporting structure of yttria doped tetragonal zirconia (YTZP), comprised of yttria doped tetragonal zirconia ceramic having a grain size, as measured by the linear intercept method, of less than 0.6 µm, wherein the pressing glass has a thermal expansion coefficient (TEC) of between 9.0 and 11.0 µm/m.K (measured in the range of from 25 to 500°C) and wherein the pressing glass has a pressing temperature of between 750 and 1000°C.

2. The process according to claim 1, wherein the supporting structure is comprised of densely sintered ceramic produced by CAD/CAM technology.

3. The process according to claim 2, wherein the ceramic is milled by a CAD/CAM-system in the green state or in a partially sintered state, and subsequent sintering to full density.

4. The process according to claim 1 or claim 2, wherein the supporting structure is milled out of a hot isostatically hopped zirconia.

5. The process according to claim 1, wherein the supporting structure is formed by electrophoretic deposition of zirconia from a slurry, followed by sintering to full density.

6. The process according to claim 1, wherein the structure is kept short by 0.5 to 2.0 mm and the pressing glass is forming an aesthetically pleasing shoulder without showing the core at the margin.

7. The process according to any one of the claims 1-6, wherein a liner is applied to the supporting zirconia structure with a melting point less that 50°C lower than the pressing temperature of the pressing glass.

8. The process according to any one of claims 1-7, wherein the connector to the press pellet reservoir and the specimen is a continuous flow plate having a thickness of 1.5-2.5 mm.

9. Full-ceramic dental restoration, comprising a fully or partially supporting structure of yttria doped tetragonal zirconia (YTZP) and a heat pressed tooth coloured pressing glass, obtainable by the process of any one of the claims 1-8.

## Patentansprüche

1. Verfahren zur Herstellung einer Vollkeramikzahnrestaurierung, umfassend das Warmpressen eines zahnfarbenen Glaspresslings auf eine vollständige oder teilweise Trägerstruktur aus mit Yttriumoxid dotiertem tetragonalem Zirkoniumdioxid (YTZP), umfassend eine mit Yttriumoxid angereicherte tetragonale Zirkoniumdioxidkeramik mit einer durch das lineare Abschnittverfahren gemessenen Korngröße von weniger als 0,6 µm, wobei der Glaspressling einen Wärmeausdehnungskoefiizienten (TEC) zwischen 9,0 und 11.0 µm/m.K (gemessen im Bereich von 25 bis 500°C) aufweist und wobei der Glaspressling eine Presstemperatur zwischen 750 und 1000°C aufweist.

2. Verfahren nach Anspruch 1, wobei die Trägerstruktur aus einer durch CAD/CAM-Technologie hergestellten dicht gesinterten Keramik zusammengesetzt ist.

3. Verfahren nach Anspruch 2, wobei die Keramik in ungesintertem Zustand oder in teilweise gesintertem Zustand durch ein CAD/CAM-System gemahlen und anschließend zur vollen Dichte gesintert wird.

4. Verfahren nach Anspruch 1 oder Anspruch 2, wobei die Trägerstruktur aus einem heißen isostatisch gehoppten Zirkoniumdioxid heraus gemahlen wird.

5. Verfahren nach Anspruch 1, wobei die Trägerstruktur durch elektrophoretische Abscheidung von Zirkoniumdioxid aus einer Aufschlämmung, gefolgt von Sintern zu voller Dichte gebildet wird.

6. Verfahren nach Anspruch 1, wobei die Struktur mit 0,5 bis 2,0 mm kurz gehalten wird und der Glaspressling eine ästhetisch angenehme Schulter bildet, ohne dass der Kern am Rand zu sehen ist.

7. Verfahren nach einem der Ansprüche 1-6, wobei eine Zwischenlage mit einem um 50°C niedrigeren Schmelzpunkt als die Presstemperatur des Glaspresslings auf der Trägerzirkoniumdioxidstruktur aufgebracht ist.

8. Verfahren nach einem der Ansprüche 1-7, wobei das Anschlussstück an den Presspelletbehälter und die Probe eine kontinuierliche Fließplatte mit einer Dicke von 1,5-2,5 mm ist.

9. Vollkeramikzahnrestaurierung, umfassend eine vollständige oder teilweise Trägerstruktur aus mit Yttriumoxid dotiertem tetragonalem Zirkoniumdioxid (YTZP) und einen heissgepressten zahnfarbenen Glaspressling, erhältlich gemäß dem Verfahren nach einem der Ansprüche 1-8.

## Revendications

1. Procédé pour la préparation d'une restauration dentaire entièrement en céramique, comprenant le pressage à chaud d'un verre à presser coloré dentaire sur une structure support entière ou partielle de zircone tétragonale dopée à l'oxyde d'yttrium (YTZP), comprenant de la céramique de zircone tétragonale dopée à l'oxyde d'yttrium ayant une grosseur de grain, telle que mesurée par le procédé d'interception linéaire, de moins de 0,6 µm, dans lequel le verre à presser a un coefficient de dilatation thermique (TEC) compris entre 9,0 et 11,0 µm/m.K (mesuré dans la gamme de 25 à 500°C) et dans lequel le verre à presser a une température de pressage comprise entre 750 et 1000°C.

2. Procédé selon la revendication 1, dans lequel la structure support est constituée de céramique densément frittée produite par la technologie CAO/FAO.

3. Procédé selon la revendication 2, dans lequel la céramique est moulue grâce à un logiciel CAO/FAO dans l'état vert ou dans un état partiellement fritté, et le frittage subséquent jusqu'à la densité maximale.

4. Procédé selon la revendication 1 ou la revendication 2, dans lequel la structure support est moulue à partir d'une zircone pressée isostatiquement à chaud.

5. Procédé selon la revendication 1, dans lequel la structure support est formée par dépôt par électrophorèse de la zircone à partir d'une boue, suivie de frittage jusqu'à la densité maximale.

6. Procédé selon la revendication 1, dans lequel la structure support est raccourcie de 0,5 à 2,0 mm et le verre pressé forme un épaulement esthétiquement agréable sans montrer le coeur au bord.

7. Procédé selon l'une quelconque des revendications 1 à 6, dans lequel un fond protecteur est appliqué à la structure support en zircone avec un point de fusion de moins de 50°C plus faible que la température de pressage du verre à presser.

8. Procédé selon l'une quelconque des revendications 1 à 7, dans lequel le connecteur entre le réservoir de pastilles de la presse et le spécimen est un plateau à flux continu ayant une épaisseur de 1,5 - 2,5 mm.

9. Restauration dentaire complète en céramique, comprenant une structure support entière ou partielle de zircone tétragonale dopée à l'oxyde d'yttrium (YTZP) et un verre à presser coloré dentaire pressé à chaud que l'on peut obtenir par le procédé selon l'une quelconque des revendications 1-8.
